Europäisches Patentamt

European Patent Office

Office européen des brevets

(11) Publication number: **0 406 454 A1**

# EUROPEAN PATENT APPLICATION

(21) Application number: 89112101.4

(51) Int. Cl.⁵: **A61N 5/06**

(22) Date of filing: 03.07.89

(43) Date of publication of application:
09.01.91 Bulletin 91/02

(84) Designated Contracting States:
AT BE DE ES FR GB GR IT LU NL SE

(71) Applicant: **TECLAS TECNOLOGIE LASER SA**
**Zona Industriale**
**CH-6594 Contone(CH)**

(72) Inventor: **Albini, Domenico**

Casa Brunella
CH-6594 Contone(CH)
Inventor: **Blandamura, Francesco**
**Via Cristoforo Colombo 3**
**I-10128 Torino(IT)**

(74) Representative: **Pozzoli, Giuliano**
**e/o ENGIMPEX S.A. Industrial Property**
**Services Dept. P.O. Box 12**
**CH-6924 Lugano-Sorengo(CH)**

(54) Lamp for photochemoterapy.

(57) A halogen source (1) emits a light whose radiations are filtered (2) and condensed (3) in order to be channelized on fiber optics (4) of a diagnostic/ therapeutic probe (4′) with selection (2′) of the band of emission.

The concomitant use of suitable pharmacological preparations, particularly porphyrins, phtalocyanines and their derivatives with photosensitive characteristics, allows to perform the therapy of neoplastic cells.

Therefore it is avoided the use of dye-laser sources of considerable manufacturing cost and smaller operational flexibility.

EP 0 406 454 A1

Fig. 1

## LAMP FOR PHOTOCHEMOTHERAPY

The present invention refers to a lamp for photochemotherapy. It represents an alternative source for the photochemotherapy.

It is well known that the photochemotherapy represents a therapeutic method which allows to cause a selective damage to the neoplastic cells under the condition that these have retained a sufficient amount of photosensitive drug.

The photosensitization requires usually moderate intensity of light with well defined wave lengths.

Up to the present there is not yet any drug which can be defined perfect, even if the results obtained till now lead to the conclusion that the photochemotherapy is a good way to follow for the therapy of the tumours.

The methodology requires to have, besides the drug, a suitable source of light; the source which has been used till now is the expensive dye-laser.

The present invention refers to an alternative source of light, suitable to this therapy, which presents the following main peculiarities:

- Low production cost, substantially lower than the cost of the dye-laser sources
- easy handling
- high versatility, allowing the test with different types of drugs
- besides the therapy of the tumours, it allows their diagnosis too.

The lamp for photochemotherapy (PTL) is the result of a cooperation between the Institute of Biology of the University of Padova and the scientific department of Teclas SA, Contone, in accordance with the existing control standards for the photochemotherapy of the tumours, approved by different Health Authorities.

The lamp allows the chemotherapeutic treatment of the cellular hyperproliferation, when it is associated to organic dyes like porphyrins, phtalocyanines and their derivatives.

The equipment represents an effective, and at the same time cheap, alternative to the use of the laser and it is used for the superficial treatment and for the treatment of the external cavities.

More precisely the present invention refers to a lamp for photochemotherapy and diagnostic characterized by the fact that it includes a halogen photoradiating source connected to a transformer with the interposition of analogue means of control of the output power and of compensation of the same; athermic filters and lens for radiation condensation; set of filters for the specific application; very efficient means for channelizing the radiation beam.

We give now in the following a not-limitative description of a preferred form of execution of the object of the invention, with reference to the figures of the attached drawings, without depriving anything from the generality of the invention itself.

- The figure 1 represents a diagram of the parts and a functional diagram of the lamp object of the invention
- The figures 2, 2 bis and 2 ter describe the therapeutic effect of the PTL radiation on a rat.
- The figures 3, 3 bis and 3 ter illustrate the therapeutic effect of the PTL on a basalioma on the nose of a human subject.
- The figures 4, 4 bis, 4 ter and 4 quater illustrate the therapeutic effect of the treatment by means of the PTL for a sarcoma of Kaposi on a sick person suffering from this disease.

The photochemotherapeutic lamp is characterized by the fact that the emission is produced by a broadband halogen lamp. The irradiation happens by means of a fiber optics beam. The desidered band is selected using the suitable filters. The duration of the treatment can be pre-arranged by means of a timer.

Technical data:

| Band power | mW 1000 |
| Electrical absorption | VA max 400 |
| Dimensions | mm 400x275x215 |
| Weight | kg 14 |
| Length of fiber optics | m 1 |
| Diameter of fiber optics | mm 8 |

Advantages:

- Easy handling
- Easy transportation
- Adequate length and high flexibility of the fiber optics beam
- Filters for different dyes and therefore different wave lengths, interchangeable by means of panels.
- Effective alternative to the laser at appreciably reduced costs.

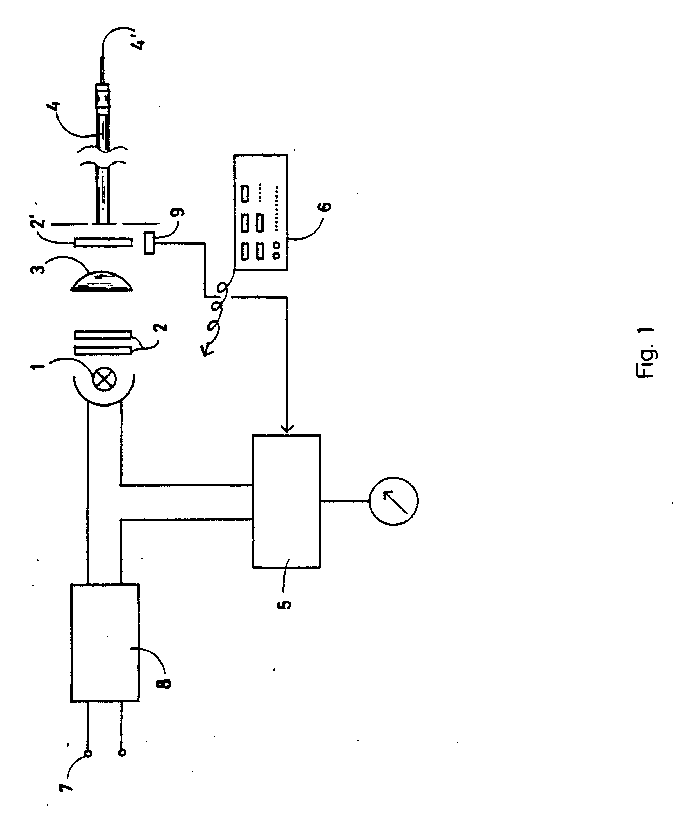

The equipment (fig. 1) consists essentially of a halogen lamp (1), whose radiation is opportunely filtered by athermic filters (2) to eliminate the undesidered emissions and connected to the electric network (8) by means of a transformer (7) with the interposition of an analogue system (5) of control of the output power acting also as a compensator.

An optical condenser (3) allows to collect in a fiber optics beam (4) the required emission bands, channelized in a diagnostic/therapeutic probe (4'). A set of filters (2') for optional applications selects the emission band and is compensated by means of the photoelectric cell (9) by the analogue device (5). An electronic/mechanic control system (6) allows to vary the emission band and its power and duration, in order to adapt the object to the different therapeutic needs.

It must be noted that it is possible to adapt commercial halogen sources devoted to other use in the equipment object of the invention.

The control system (6) allows the following control/monitoring functions to be performed:
- Switching-on
- Input for the diagnostic
- Survey of the diagnostic data
- Input for the therapy
- Control of the therapy functions
- Timer
- Operation/Failure control lights
- Safety devices and safety checks
- Switching-off

These functions and the displays can be possibly digitized.

The more usual applications of the PTL refer to all the pathologies characterized by the hyperproliferation of cells or tissue:
solid tumours - atheromatous plates - serous gemmation from neoplasia - microbial infections - psoriatic lesions.

Particularly it has been successfully used for the treatment of the male and female external genitals and in the oral cavity.

The association between the surgery and the chemotherapy has proved itself particularly useful in the case of cerebral tumours.

The novelty of the invention resides then substantially in the use in therapeutic devices of the radiation of usual halogen lamps, even of commercial type, exploiting their photoradiating effect of destruction of the sick cells in concomitance with the chemical attack in the presence of suitable photosensitive pharmacological preparations.

It is used particularly in the photodynamic therapy of the adenoma, of the cutaneous or subcutaneous superficial tumours.

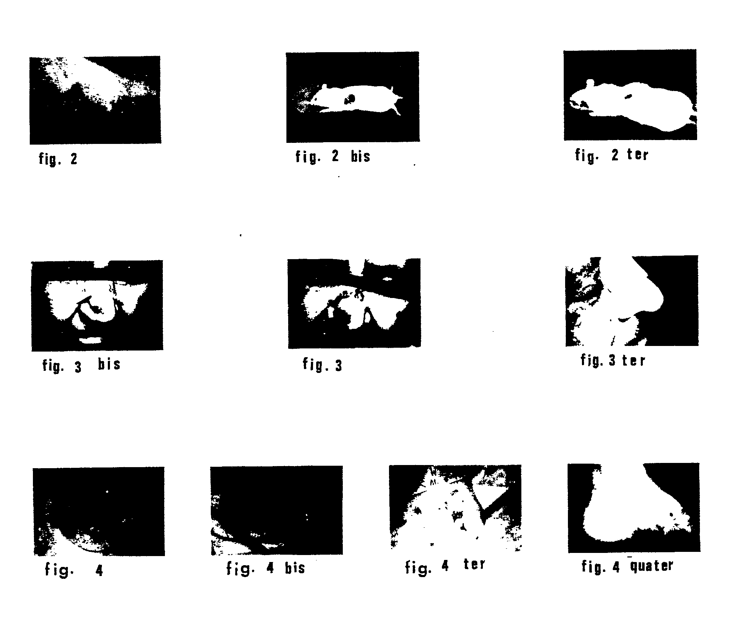

EXAMPLE 1 (figures 2, 2 bis, 2 ter)

The figures show in succession the therapeutic effect associated to the PTL radiation with power density of 300 J/cm² on a rat suffering from hepatoma of Yoshida respectively before the treatment, after 5 days and after 15 days.

EXAMPLE 2 (figures 3, 3 bis, 3 ter)

The figures describe in succession the therapeutic effect associated to the PTL radiation with power density of 80 J/cm² on a human subject suffering from a basalioma on the nose respectively before the treatment, after the first treatment and after a further treatment performed after 24 hours.

EXAMPLE 3 (figures 4, 4 bis, 4 ter, 4 quater)

The figures illustrate in succession the therapeutic effect in the human being associated to the PTL radiation with power density of 60-70 J/cm² on a patient suffering from sarcoma of Kaposi, respectively before the treatment, after 4 months, after 8 months and after 1 year.

## Claims

1) Lamp for photochemotherapy and diagnostic characterized by the fact that it includes a halogen photoradiating source (1) connected to a transformer (8) with the interposition of analogue means of control of the output power and of compensation of the same; athermic filters (2) and lens (3) for radiation condensation; set of filters (2') for the specific application; very efficient means (4, 4') for channelizing the radiation beam.

2) Lamp according to the Claim 1, where the halogen source (1) is an usual commercial one.

3) Lamp according either to the Claim 1 or to the Claim 2, where the channelizing mean of the radiation beam (4, 4') is a fiber optics probe.

4) Equipment for photochemotherapy including the lamp according to one of the Claims 1 to 3, associated to accessories like interchangeable filters and/or light delivery devices and photosensitive drugs for specific use.

5) Equipment according to the Claim 4, where the photosensitive drugs are organic dyes, like porphyrins, phtalocyanines and their derivatives and similars.

Fig. 1

fig. 2

fig. 2 bis

fig. 2 ter

fig. 3 bis

fig. 3

fig. 3 ter

fig. 4

fig. 4 bis

fig. 4 ter

fig. 4 quater

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | EP-A-46939 (DENTSPLY INTERNATIONAL INC) <br> * page 3, line 29 - page 4, line 22 * <br> * page 5, lines 3 - 18 * <br> * page 5, line 34 - page 11, line 5 * <br> --- | 1-5 | A61N5/06 |
| Y | DE-A-3323365 (DIETRICH) <br> * pages 5,6 - 9-12 * <br> --- | 1-5 | |
| A | WO-A-8301311 (CHAPMAN) <br> * page 5-7 * <br> ----- | 1, 3-5 | |
| | | | TECHNICAL FIELDS SEARCHED (Int. Cl.5 ) |
| | | | A61N |

The present search report has been drawn up for all claims

| Place of search | Date of completion of the search | Examiner |
|---|---|---|
| THE HAGUE | 06 MARCH 1990 | LEMERCIER D.L.L. |